# EUROPÄISCHE PATENTSCHRIFT

(11) **EP 0 807 615 B1**
(45) Veröffentlichungstag und Bekanntmachung des Hinweises auf die Patenterteilung: **18.12.2002**
(21) Anmeldenummer: 97107496.8
(22) Anmeldetag: 07.05.1997
(51) Int. Cl.: C04B 35/14, B01J 21/08, B01J 35/10, B01J 37/00, C09C 1/30

(54) **Presslinge auf Basis von pyrogen hergestelltem Siliciumdioxid**
Pressed articles of pyrogenically prepared silicon dioxide
Comprimé à base de SiO2 preparé par pyrogénation

(30) Priorität: 17.05.1996 DE 19619961
(43) Veröffentlichungstag der Anmeldung: 19.11.1997
(73) Patentinhaber: Degussa AG, 40474 Düsseldorf (DE)
(72) Erfinder: Krause, Helmfried, Dr., 63517 Rodenbach (DE); Lansink-Rotgerink, Hans, Dr., 63864 Glattbach (DE); Tacke, Thomas, Dr., 61381 Friedrichsdorf (DE); Panster, Peter, Dr., 63517 Rodenbach (DE); Burmeister, Roland, Dr., 63825 Geiselbach (DE)

(56) Entgegenhaltungen:
- EP-A- 0 725 037
- DE-A- 3 912 504
- DE-A- 4 427 574
- DE-C- 3 803 895
- DE-C- 3 803 899

## Beschreibung

Die Erfindung betrifft Preßlinge auf Basis von pyrogen hergestelltem Siliciumdioxid, ein Verfahren zu ihrer Herstellung und ihre Verwendung als Katalysatorträger oder Katalysator.

Pyrogen hergestellte Siliciumdioxide zeichnen sich durch extreme Feinteiligkeit und entsprechend hohe spezifische Oberfläche, sehr hohe Reinheit, sphärische Teilchenform und das Fehlen von Poren aus. Aufgrund dieser Eigenschaften finden die pyrogen hergestellten Oxide zunehmend Interesse als Träger für Katalysatoren (D. Koth, H. Ferch, Chem. Ing. Techn. 52, 628 (1980)).

Da pyrogen hergestellte Oxide besonders feinteilig sind, bereitet die Verformung zu Katalysatorträgern bzw. Katalysatoren einige Schwierigkeiten.

Aus der DE-A 31 32 674 ist ein Verfahren zur Herstellung von Preßlingen aus pyrogen hergestellten Oxiden bekannt, bei welchem Kieselsol als Bindemittel verwendet wird.

Aus der DE-A 34 06 185 ist ein Verfahren zur Herstellung von Preßlingen bekannt, bei welchem man Glasurfrittenpulver als Bindemittel und Glycerin als Gleitmittel verwendet.

Aus der DE-B 21 00 778 ist bekannt, Granulate auf Basis pyrogen hergestellter Siliciumdioxide bei der Herstellung von Vinylacetatmonomer als Katalysatorträger einzusetzen.

Aus der DE-A 39 12 504 ist ein Verfahren zur Herstellung von Preßlingen bekannt, bei dem man Aluminiumstearat, Magnesiumstearat und/oder Graphit als Gleitmittel und Harnstoff sowie Methylcellulose als Porenbildner verwendet.

Diese bekannten Preßlinge werden als Aerosil-Tabletten Nr. 350, Firma Degussa, in den Handel gebracht. Sie weisen ca. 0,4 Gew.-% Mg auf.

Aus der EP-B 0 519 435 ist es bekannt, SiO₂ mittels Bindemittel zu Träger zu verpressen, die erhaltenen Träger zu glühen und die geglühten Trägerteilchen mittels Säure zu waschen, bis keine weiteren Kationen des Bindemittels abgegeben werden.

Die bekannten Verfahren haben den Nachteil, daß die erhaltenen Preßlinge für bestimmte katalytische Reaktionen, wie zum Beispiel die Vinylacetatherstellung aus Ethylen, Essigsäure und Sauerstoff oder die Hydratisierung von Ethylen zu Ethanol, nicht die gewünschten optimalen Eigenschaften, wie zum Beispiel eine hohe Reinheit, hohe Aktivität, hohe Selektivität, hohe Produktausbeute und hohe Stabilität aufweisen.

Gegenstand der Erfindung sind Preßlinge auf Basis von pyrogen hergestelltem Siliciumdioxid mit den folgenden physikalisch-chemischen Kenndaten:

| | |
|---|---|
| Außendurchmesser | 0,8 - 20 mm |
| BET-Oberfläche | 30 - 400 m²/g |
| Porenvolumen | 0,5 - 1,3 ml/g |
| Bruchfestigkeit | 10 bis 250 N |
| Zusammensetzung | > 99,8 Gew.-% SiO₂ |
| Sonstige Bestandteile | < 0,2 Gew.-% |
| Abrieb | < 5 Gew.-% |
| Schüttgewicht | 350 - 750 g/l |

Ein weiterer Gegenstand der Erfindung ist ein Verfahren zur Herstellung von Preßlingen auf Basis von pyrogen hergestelltem Siliciumdioxid mit folgenden physikalisch-chemischen Kenndaten:

| | |
|---|---|
| Außendurchmesser | 0,8 - 20 mm |
| BET-Oberfläche | 30 - 400 m²/g |
| Porenvolumen | 0,5 - 1,3 ml/g |
| Bruchfestigkeit | 10 - 250 N |
| Zusammensetzung | > 99,8 Gew.-% SiO₂ |
| Sonstige Bestandteile | < 0,2 Gew.-% |
| Abrieb | < 5 Gew.-% |
| Schüttgewicht | 350 - 750 g/l |

welches dadurch gekennzeichnet ist, daß man pyrogen hergestelltes Siliciumdioxid mit Methylcellulose, Mikrowachs und/oder Polyethylenglycol unter Zusatz von Wasser homogenisiert, bei einer Temperatur von 80 - 150 °C trocknet, gegebenenfalls zu einem Pulver zerkleinert, das Pulver zu Preßlingen verpreßt und während eines Zeitraumes von 0,5 bis 8 Stunden bei einer Temperatur von 400 bis 1200 °C tempert.

Zur Durchführung des erfindungsgemäßen Verfahrens sind alle Mischer oder Mühlen, die eine gute Homogenisierung ermöglichen, wie zum Beispiel Schaufel-, Wirbelschicht-, Kreisel- oder Luftstrommischer, geeignet. Besonders geeignet sind Mischer, mit denen eine zusätzliche Verdichtung des Mischgutes möglich ist, wie zum Beispiel Pflugscharmischer, Kollergänge oder Kugelmühlen. Nach dem Homogenisieren kann eine weitgehende Trocknung bei 80 - 150 °C erfolgen, so daß man nach gegebenenfalls durchgeführtem Zerkleinern ein rieselfähiges Pulver erhält. Die Herstellung der Preßlinge kann auf Stempelpressen, Exzenterpressen, isostatischen Pressen, Strangpressen oder Rundlaufpressen als auch auf Kompaktoren erfolgen.

Vor dem Verpressen kann in einer besonderen Ausführungsform der Erfindung die Mischung die folgende Zusammensetzung aufweisen:
50 - 90 Gew.-% Siliciumdioxid, vorzugsweise 65 - 85 Gew.-%
0,1 - 20 Gew.-% Methylcellulose, vorzugsweise 5 - 15 Gew.-%
0,1 - 15 % Mikrowachs, vorzugsweise 5 - 10 Gew.-%
0,1 - 15 % Polyethylenglykol, vorzugsweise 5 - 10 Gew.-%

Die Preßlinge können verschiedene, zum Beispiel zylinderische, kugelförmige oder ringförmige Formen mit einem Außendurchmesser von 0,8 bis 20 mm aufweisen. Die Preßlinge werden bei 400 - 1200 °C 30 Minuten bis 8 Stunden getempert. Durch Variation der Einsatzstoffmengen und des Preßdruckes können die Bruchfestigkeit, die spezifische Gesamtoberfläche und das Porenvolumen in einem gewissen Rahmen eingestellt werden.

Die erfindungsgemäßen Preßlinge können entweder direkt als Katalysator oder als Katalysatorträger eingesetzt werden. In dem letzten Fall können die Preßlinge nach ihrer Herstellung mit einer katalytisch wirksamen Substanz in Kontakt gebracht und gegebenenfalls durch eine geeignete Nachbehandlung aktiviert werden.

Insbesondere lassen sich die Preßlinge aus pyrogen hergestelltem Siliciumdioxid als Träger für den Katalysator bei der Herstellung von Vinylacetatmonomer aus Ethylen, Essigsäure und Sauerstoff sowie als Katalysatorträger im Olefinhydratisierungsverfahren verwenden.

Die erfindungsgemäßen Preßlinge weisen die folgenden Vorteile auf:

Gegenüber den Preßlingen gemäß dem Dokument DE-A 39 12 504 weisen die erfindungsgemäßen Preßlinge außer Siliciumdioxid keine anderen anorganischen Bestandteile auf. Die bekannten Preßlinge haben den Nachteil, daß sie ca. 0,4 Gew.-% Mg enthalten, welches während des Verfahrens zur Hydratisierung von Olefinen ausgelaugt wird.

Die erfindungsgemäßen Preßlinge weisen dagegen eine verbesserte hydrothermale Stabilität bei derartigen Hydratisierungsverfahren auf. Zusätzlich weisen sie eine hohe Reinheit und ein hohes Porenvolumen auf.

Ein weiterer Gegenstand der Erfindung ist ein Trägerkatalysator für die Produktion von Vinylacetatmonomer (VAM), welcher auf dem erfindungsgemäßen Träger aus Siliziumdioxid als katalytisch aktive Komponenten Palladium, Gold und Alkaliverbindungen enthält, sowie ein Verfahren zu seiner Herstellung und seine Verwendung.

Gold, Palladium und Alkaliverbindungen enthaltende Trägerkatalysatoren werden für die Produktion von Vinylacetat verwendet. Dazu werden Ethen, Essigsäure und molekularer Sauerstoff bzw. Luft in der Gasphase, gegebenenfalls unter Zusatz von Inertgasen, bei Temperaturen zwischen 100 und 250 °C und gewöhnlichem oder erhöhtem Druck in Gegenwart des Trägerkatalysators zur Reaktion gebracht.

Ein derartiges Produktionsverfahren ist aus den Dokumenten DE 16 68 088, US 4,048,096 und EP 0 519 435 B1 bekannt. Diese Patentschriften offenbaren auch ein Verfahren zur Herstellung der Gold, Palladium und Alkaliverbindungen enthaltenden Trägerkatalysatoren. Je nach Ausführungsform werden Katalysatoren mit homogener Edelmetallverteilung über den Trägerquerschnitt und mit mehr oder weniger ausgeprägtem Schalenprofil erhalten.

Diese Katalysatoren werden gewöhnlich durch Imprägnieren der Träger mit einer basischen Lösung und einer Gold- und Palladiumsalze enthaltenden Lösung erhalten, wobei die Imprägnierschritte gleichzeitig oder nacheinander, mit oder ohne Zwischentrocknung erfolgen. Anschließend wird der Träger zur Entfernung gegebenenfalls vorhandener Chloridanteile gewaschen. Vor oder nach dem Waschen werden die auf dem Träger ausgefällten unlöslichen Edelmetallverbindungen reduziert. Die so erhaltene Katalysatorvorstufe wird getrocknet und zur Aktivierung des Katalysators mit Alkaliacetaten oder Alkaliverbindungen imprägniert, die sich unter den Reaktionsbedingungen bei der Produktion von Vinylacetatmonomer ganz oder teilweise in Alkaliacetate umwandeln.

Die Reduktion des Katalysators kann in der wäßrigen Phase oder in der Gasphase vorgenommen werden. Zur Reduktion in der wäßrigen Phase eignen sich zu Beispiel Formaldehyd oder Hydrazin. Die Reduktion in der Gasphase kann mit Wasserstoff beziehungsweise Formiergas (95 Vol.-% N₂ + 5 Vol.-% H₂) oder Ethen vorgenommen werden. Gemäß der EP 0 634 209 erfolgt die Reduktion mit Wasserstoff bei Temperaturen zwischen 40 und 260 °C, bevorzugt zwischen 70 und 200 °C. Häufig wird der Katalysator jedoch erst nach der Aktivierung mit Alkaliacetat direkt im Produktionsreaktor mit Ethen reduziert.

Im Produktionsprozeß wird der Katalysator erst langsam mit den Reaktanden belastet. Während dieser Anfahrphase erhöht sich die Aktivität des Katalysators und erreicht gewöhnlich erst nach Tagen oder Wochen ihr endgültiges Niveau.

Aufgabe der vorliegenden Erfindung ist es, einen Trägerkatalysator für die Produktion von Vinylacetatmonomer anzugeben, welcher bei gleicher beziehungsweise verbesserter Selektivität eine höhere Aktivität als bekannte Katalysatoren aufweist.

Gegenstand der Erfindung ist ein Trägerkatalysator, welcher auf dem erfindungsgemäßen Träger aus Siliziumdioxid als katalytisch aktive Komponenten Palladium, Gold und Alkaliacetat enthält.

Als Trägermaterial für den Katalysator eignet sich der erfindungsgemäße Preßling auf Basis von pyrogen hergestelltem Siliciumdioxid. Wichtig ist, daß die Katalysatorträger unter den Reaktionsbedingungen des katalytischen Prozesses, insbesondere unter dem Einfluß von Essigsäure, ihre mechanische Festigkeit behalten.

Die erfindungsgemäßen Preßlinge können als Strangpreßlinge, Tabletten, Ringe oder in anderen für Festbettkatalysatoren üblichen Formen vorliegen.

Die erfindungsgemäßen Preßlinge werden mit einer Palladium und Gold enthaltenden Lösung imprägniert. Gleichzeitig mit der edelmetallhaltigen Lösung oder in beliebiger Reihenfolge nacheinander werden die erfindungsgemäßen Preßlinge mit einer basischen Lösung imprägniert, welche ein oder mehrere basische Verbindungen enthalten kann. Die basische Verbindung oder Verbindungen dienen zur Überführung des Palladiums und Golds in ihre Hydroxide.

Die Verbindungen in der basischen Lösung können aus Alkalihydroxiden, Alkalibicarbonaten, Alkalicarbonaten, Alkalisilikaten oder Mischungen dieser Stoffe bestehen. Bevorzugt werden Kaliumhydroxid und Natriumhydroxid verwendet.

Zur Herstellung der edelmetallhaltigen Lösung können als Palladiumsalze beispielsweise Palladiumchlorid, Natrium- bzw. Kaliumpalladiumchlorid oder Palladiumnitrat verwendet werden. Als Goldsalze eignen sich Gold(III)-chlorid und Tetrachlorogold(III)-säure. Vorzugsweise können Kaliumpalladiumchlorid beziehungsweise Natriumpalladiumchlorid und Tetrachlorogoldsäure eingesetzt werden.

Das Imprägnieren der erfindungsgemäßen Preßlinge mit der basischen Lösung beeinflußt die Abscheidung der Edelmetalle in dem Preßling. Die basische Lösung kann entweder gleichzeitig mit der Edelmetallösung oder in beliebiger Reihenfolge mit dieser Lösung mit dem erfindungsgemäßen Preßling in Kontakt gebracht werden. Bei aufeinanderfolgender Imprägnierung des erfindungsgemäßen Preßlings mit den beiden Lösungen kann nach dem ersten Imprägnierschritt eine Zwischentrocknung vorgenommen werden.

Bevorzugt wird der erfindungsgemäße Preßling zuerst mit der basischen Verbindung imprägniert. Die anschließende Imprägnierung mit der Palladium und Gold enthaltenden Lösung führt zur Ausfällung von Palladium und Gold in einer oberflächlichen Schale auf dem erfindungsgemäßen Preßling. Die umgekehrte Reihenfolge der Imprägnierungen führt im allgemeinen zu einer mehr oder weniger homogenen Verteilung der Edelmetalle über den Querschnitt des erfindungsgemäßen Preßlings. Bei geeigneter Verfahrensführung können jedoch auch bei umgekehrter Imprägnier-Reihenfolge Katalysatoren mit definierter Schale erhalten werden (siehe z. B. US 4, 048, 096). Katalysatoren mit homogener oder nahezu homogener Edelmetall-Verteilung weisen im allgemeinen eine geringere Aktivität und Selektivität auf.

Katalysatoren mit Schalendicken von unter 1 mm, bevorzugt von etwa unter 0,5 mm, sind besonders geeignet. Die Schalendicke wird durch die Menge der auf das Trägermaterial aufgebrachten basischen Verbindung relativ zur gewünschten Menge der Edelmetalle beeinflußt. Je höher dieses Verhältnis ist, desto geringer wird die Dicke der sich ausbildenden Schale. Das für eine gewünschte Schalendicke erforderliche Mengenverhältnis von basischer Verbindung zu den Edelmetallverbindungen hängt von der Beschaffenheit des Trägermaterials sowie von der gewählten basischen Verbindung und den Edelmetallverbindungen ab. Das erforderliche Mengenverhältnis wird zweckmäßigerweise durch wenige Vorversuche ermittelt. Die sich ergebende Schalendicke kann dabei in einfacher Weise durch aufschneiden der Katalysatorteilchen ermittelt werden.

Die minimal notwendige Menge der basischen Verbindung ergibt sich aus der stöchiometrisch berechneten Menge- an Hydroxidionen, die zur Überführung des Palladiums und Golds in die Hydroxide benötigt werden. Als Richtwert gilt, daß die basische Verbindung für eine Schalendicke von 0,5 mm in einem 1 bis 10-fachen stöchiometrischen Überschuß angewendet werden sollte.

Die erfindungsgemäßen Preßlinge werden nach dem Verfahren der Porenvolumenimprägnierung mit den basischen Verbindungen und den Edelmetallsalzen belegt. Wird mit Zwischentrocknung gearbeitet, wählt man die Volumina der beiden Lösungen so, daß sie jeweils etwa 90 bis 100 % der Aufnahmekapazität der erfindungsgemäßen Preßlinge entsprechen. Wird auf die Zwischentrocknung verzichtet, so muß die Summe der Einzelvolumina der beiden Imprägnierlösungen der obigen Bedingung entsprechen, wobei die Einzelvolumina im Verhältnis von 1 : 9 bis 9 : 1 zueinander stehen können. Bevorzugt wird ein Volumenverhältnis von 3 : 7 bis 7 : 3, insbesondere von 1 : 1, angewendet. Als Lösungsmittel kann in beiden Fällen bevorzugt Wasser verwendet werden. Es können aber auch geeignete organische oder wäßrig-organische Lösungsmittel eingesetzt werden.

Die Umsetzung der Edelmetallsalzlösung mit der basischen Lösung zu unlöslichen Edelmetallverbindungen erfolgt langsam und ist je nach Präparationsmethode im allgemeinen erst nach 1 bis 24 Stunden abgeschlossen. Danach werden die wasserunlöslichen Edelmetallverbindungen mit Reduktionsmitteln behandelt. Es kann eine Naßreduktion zum Beispiel mit wäßrigem Hydrazinhydrat oder eine Gasphasenreduktion mit Wasserstoff, Ethen, Formiergas oder auch Methanoldämpfen vorgenommen werden. Die Reduktion kann bei Normaltemperatur oder erhöhter Temperatur und bei Normaldruck oder erhöhtem Druck erfolgen; gegebenenfalls auch unter Zugabe von Inertgasen.

Vor beziehungsweise nach der Reduktion der Edelmetallverbindungen wird das auf dem Träger gegebenenfalls vorhandene Chlorid durch eine gründiche Waschung entfernt. Nach der Waschung sollte der Katalysator weniger als 500, besser weniger als 200 ppm, Chlorid enthalten.

Die nach der Reduktion erhaltene Katalysatorvorstufe wird getrocknet und abschließend mit Alkaliacetaten oder Alkaliverbindungen imprägniert, die sich unter den Reaktionsbedingungen bei der Produktion von Vinylacetatmonomer ganz oder teilweise in Alkaliacetate umwandeln. Vorzugsweise wird mit Kaliumacetat imprägniert. Hierbei wird wieder bevorzugt die Porenvolumenimprägnierung angewendet, das heißt, die benötigte Menge Kaliumacetat wird in einem Lösungsmittel, vorzugsweise Wasser, gelöst, dessen Volumen etwa der Aufnahmekapazität des vorgelegten Trägermaterials für das gewählte Lösungsmittel entspricht. Dieses Volumen ist etwa gleich dem Gesamtporenvolumen des Trägermaterials.

Der fertige Katalysator wird anschießend bis auf eine Restfeuchte von weniger als 2 % getrocknet. Die Trocknung kann an Luft, gegebenefalls auch unter Stickstoff als Inertgas, erfolgen.

Für die Synthese von Vinylacetatmonomer ist es zweckmäßig, den Katalysator mit 0,2 bis 4, vorzugsweise 0,3 bis 3 Gew.-% Palladium, 0,1 bis 2, vorzugsweise 0,15 bis 1,5 Gew.-% Gold und 1 bis 10, vorzugsweise 3,5 bis 10 Gew.-% Kaliumacetat, jeweils bezogen auf das Gewicht des eingesetzten Trägers, zu belegen. Im Fall von Katalysatorträgern mit einer Schüttdichte von 500 g/l entsprechen diese Konzentrationsangaben volumenbezogenen Konzentrationen von 1,0 bis 20 g/l Palladium, 0,5 bis 10 g/l Gold und 5 bis 50 g/l Kaliumacetat. Zur Anfertigung der Imprägnierlösungen werden die entsprechenden Mengen der Palladium- und Goldverbindungen in einem Volumen Wasser gelöst, welches etwa 90 bis 100 % der Wasseraufnahmekapazität des vorgelegten Trägermaterials entspricht. Ebenso wird bei der Anfertigung der basischen Lösung verfahren.

Die Erfindung betrifft weiterhin die Hydratisierung von Olefinen zu den entsprechenden Alkoholen in Gegenwart von Phosphorsäure oder einer anderen Aktivkomponente, zum Beispiel eine Heteropolysäure, als Katalysatorträger auf dem erfindungsgemäßen Preßling.

Ein solches Verfahren wird zum Beispiel in der EP 0 578 441 A2 beschrieben. Nach diesem Verfahren wird Wasser und Ethylen bei Temperaturen zwischen 225 und 280°C und Drucken zwischen 20 und 240 bar zu Ethanol umgesetzt. Dabei wird ein Wasser/Ethylen-Molverhältnis im Bereich von 0,15 bis 0,5 angewendet. Die Katalysatorbelastung, gemessen in Gramm Wasser/Ethylen-Gemisch pro Minute und Milliliter Katalysator, kann im Bereich von 0,01 bis 0,1 g/(min x ml) gewählt werden. Als Nebenprodukt wird bei dieser Reaktion Diethylether gebildet.

Die Herstellung von Isopropanol durch Hydratisierung von Propylen erfolgt unter ähnlichen Bedingungen, jedoch bei etwas verringerter Temperatur im Bereich zwischen 180 und 225°C. Als Nebenprodukt entsteht bei dieser Reaktion n-Propanol.

Als Katalysatorträger für die Aktivkomponente Phosphorsäure können gemäß EP 0 578 441 A2 Pellets aus synthetischem Siliziumdioxid mit hoher Bruchfestigkeit, hoher Porosität und geringen metallischen Verunreinigungen eingesetzt werden. Die Poren des Trägers dienen zur Aufnahme der Aktivkomponente. Der mittlere Porenradius vor dem Einsatz im Hydratisierungsprozeß liegt im Bereich zwischen 1 und 50 nm.

Katalysatoren unterliegen im Betrieb einer Alterung, die sich durch eine Verringerung der Aktivität und/oder Selektivität bemerkbar macht. Die Desaktivierung beruht häufig auf einer durch hohe Temperaturen verursachten Verringerung der spezifischen Oberfläche des Trägers.

Die spezifische Oberfläche des Trägers ist eng mit seiner Porenstruktur verbunden. Darüber hinaus weisen hochoberflächige Feststoffe meist eine vollständig amorphe oder überwiegend amorphe Struktur auf, welche das Bestreben hat, unter Kristallitwachstum und Verringerung der spezifischen Oberfläche in einen thermodynamisch stabilen Zustand überzugehen.

Es hat sich gezeigt, daß auch Siliciumdioxid enthaltende Katalysatorträger einer solchen Alterung unterliegen.

Hydratisierungsbedingungen beschleunigen die Alterung. Weiterhin ist es bekannt, daß auch Verunreinigungen, insbesondere durch Alkalimetalle, die Alterung von Siliziumdioxid enthaltenden Trägern unter hydrothermalen Bedingungen fördern (siehe zum Beispiel R.K. Iler in "The Chemistry of Silica", Seite 544, John Wiley & Sons (1979)).

Auch die in der EP 0 393 356 beschriebenen Katalysatorträger auf Basis von pyrogen hergestelltem Siliziumdioxid unterliegen unter hydrothermalen Bedingungen einer Alterung, wobei kleine Poren zu größeren Poren unter Verlust von spezifischer Oberfläche zusammenwachsen. Das Porenvolumen ändert sich dabei anfänglich kaum.

Aufgabe der vorliegenden Erfindung ist es daher, Siliziumdioxid enthaltende Katalysatorträger anzugeben, welche bei Verwendung unter hydrothermalen Bedingungen eine verbesserte Alterungsstabilität aufweisen.

Diese Aufgabe wird gelöst durch die Verwendung von Katalysatoren, welche eine Aktivkomponente auf einem dem erfindungsgemäßen Preßling aufweisen.

Die erfindungsgemäße Verwendung ist besonders vorteilhaft für die Hydratisierung von Olefinen. Die Stabilisierung des Trägers ist jedoch auch bei anderen katalytischen Umsetzungen unter hydrothermalen Bedingungen günstig.

Im Falle der Hydratisierung von Olefinen wird Phosphorsäure als Aktivkomponente in den Katalysatorträger eingebracht. Hierzu wird der Träger in eine wäßrige Lösung von Phosphorsäure getaucht und mit dieser getränkt. Zur Anwendung kommen hierbei Phosphorsäurelösungen mit 15 bis 85 Gew.-% Phosphorsäure bezogen auf das Gesamtgewicht der Lösung.

Ein Hauptanwendungsgebiet der Hydratisierung von Olefinen ist die Hydratisierung von Ethylen zur Herstellung von Ethanol und Diethylether sowie die Hydratisierung von Propylen zur Herstellung von Isopropanol. Es werden dabei die aus dem Stand der Technik bekannten Reaktionsbedingungen angewendet.

Im folgenden wird die Änderung der Porenstruktur von Siliziumdioxid enthaltenden Katalysatorträgern unter hydrothermalen Bedingungen untersucht. Es wird ein bekannter Träger mit einem erfindungsgemäßen Träger verglichen.

Es zeigen
- Figur 1:: Porenstruktur eines Mg-enthaltenden Katalysatorträgers nach hydrothermalem Alterungstest.
- Figur 2:: Porenstruktur eines erfindungsgemäßen Katalysatorträgers nach hydrothermalem Alterungstest.

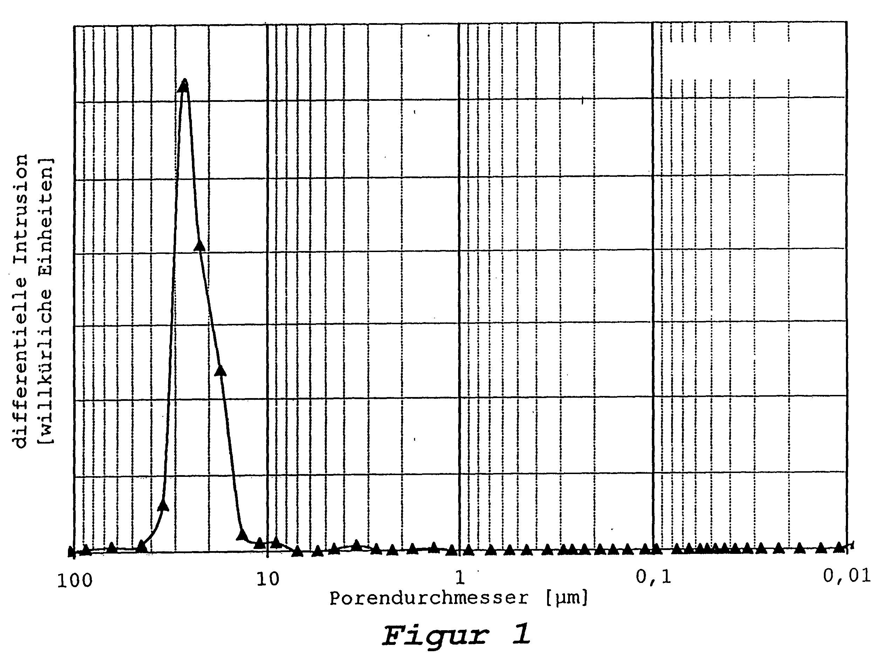

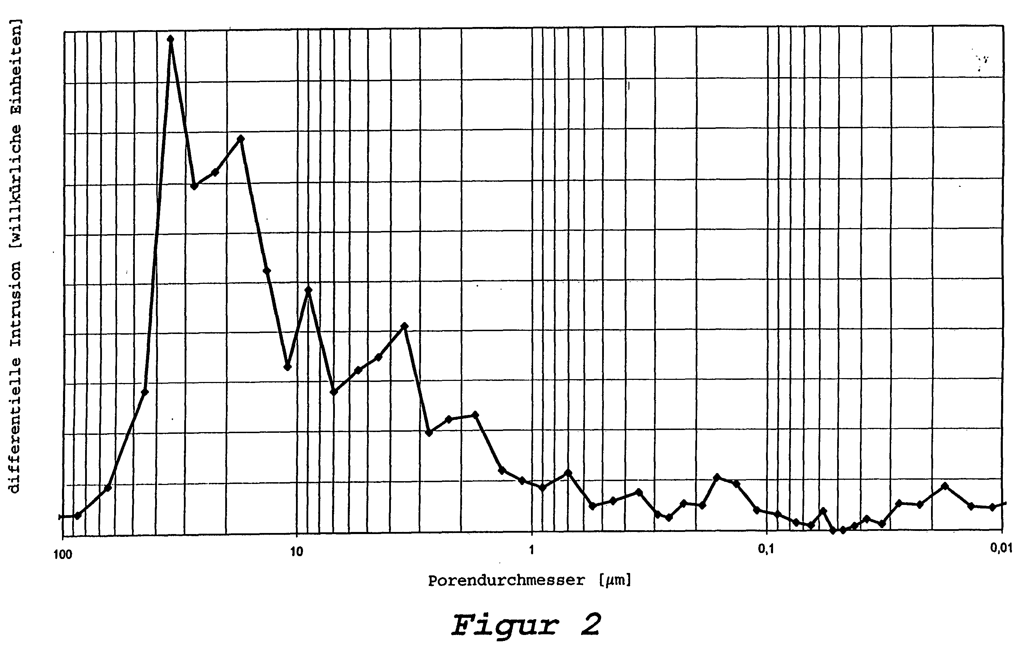

Die in den Figuren 1 und 2 gezeigten Porenverteilungskurven werden mit Hilfe der bekannten Hg-Porosimetrie ermittelt. Sie geben die differentielle Eindringung (Intrusion) des Quecksilbers in Abhängigkeit vom Porendurchmesser wieder. Für die differentielle Intrusion werden willkürliche Einheiten gewählt und die Kurven jeweils über den zur Verfügung stehenden Diagrammbereich gedehnt.

Als pyrogen hergestelltes Siliciumdioxid können Siliciumdioxide mit den folgenden physikalisch-chemischen Kenndaten eingesetzt werden:

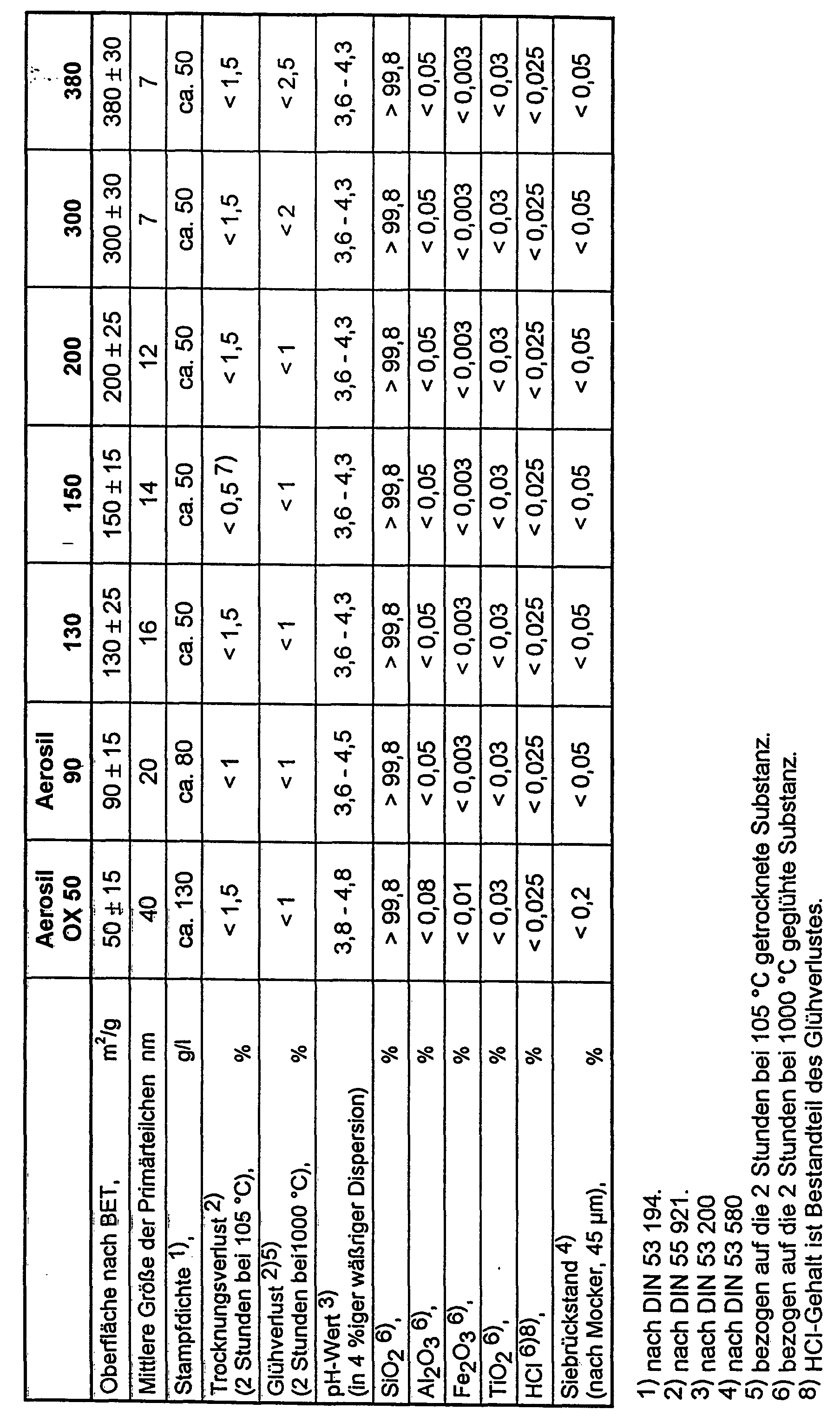

Zur Herstellung von AEROSIL wird in eine Knallgasflamme aus Wasserstoff und Luft eine flüchtige Siliciumverbindung eingedüst. In den meisten Fällen verwendet man Siliciumtetrachlorid. Diese Substanz hydrolysiert unter dem Einfluß des bei der Knallgasreaktion entstehenden Wassers zu Siliciumdioxid und Salzsäure. Das Siliciumdioxid tritt nach dem Verlassen der Flamme in eine sogenannte Koagulationszone ein, in der die AEROSIL-Primärteilchen und -Primäraggregate agglomerieren. Das in diesem Stadium als eine Art Aerosol vorliegende Produkt wird in Zyklonen von den gasförmigen Begleitsubstanzen getrennt und anschließend mit feuchter Heißluft nachbehandelt. Durch dieses Verfahren läßt sich der Rest-Salzsäuregehalt unter 0,025 % senken. Da das AEROSIL am Ende dieses Prozesses mit einer Schüttdichte von nur ca. 15 g/l anfällt, wird eine Vakuumverdichtung, mit der sich Stampfdichten von ca. 50 g/l und mehr einstellen lassen, angeschlossen.

Die Teilchengrößen der auf diese Weise gewonnenen Produkte können mit Hilfe der Reaktionsbedingungen, wie zum Beispiel die Flammentemperatur, der Wasserstoff- oder Sauerstoffanteil, die Siliciumtetrachloridmenge, die Verweilzeit in der Flamme oder die Länge der Koagulationsstrecke, variiert werden.

Die BET-Oberfläche wird gemäß DIN 66 131 mit Stickstoff bestimmt. Das Porenvolumen wird rechnerisch aus der Summe von Mikro-, Meso- und Makroporenvolumen bestimmt. Die Bruchfestigkeit wird mittels des Bruchfestigkeitstesters der Firma Erweka, Typ TBH 28, bestimmt.

Die Bestimmung der Mikro- und Mesoporen erfolgt durch Aufnahme einer N₂-Isotherme und deren Auswertung nach BET, de Boer und Barret, Joyner, Halenda. Die Bestimmung der Makroporen erfolgt durch das Hg-Einpreßverfahren.

Der Abrieb wird mittels des Abrieb- und Friabilitätstesters der Firma Erweka, Typ TAR, bestimmt.

### Beispiele 1 - 5

71,5 Gew.-% Aerosil 200
13 Gew.-% Methylcellulose
7 Gew.-% Mikrowachs
8,5 Gew.-% Polyethylenglycol
werden unter Zusatz von Wasser kompaktiert, bei 110 °C 16 Stunden getrocknet, zu einem rieselfähigen Pulver zerkleinert und mit einer Excenterpresse zu Preßlingen verformt. Die Rohtabletten werden 6 Stunden bei 750 °C calciniert.

Die erhaltenen Preßlinge weisen folgende physikalischchemische Kenndaten auf:

| **Beispiel** | **1** | **2** | **3** | **4** | **5** |
|---|---|---|---|---|---|
| Tablettenform | Zylinder | Zylinder | Zylinder | Zylinder | Ringe |
| Außendurchmesser x Höhe x Innendurchmesser (mm) | 3 x 3 | 4 x 4 | 5 x 5 | 5 x 5 | 9 x 5 x 3 |
| BET-Oberfläche (m²/g) | 170 | 164 | 168 | 163 | 165 |
| Porenvolumen (ml/g) | 0,75 | 0,84 | 0,84 | 0,97 | 0,79 |
| Bruchfestigkeit (N) | 49 | 42 | 60 | 31 | 22 |
| Abrieb (Gew.-%) | 0,9 | 1,6 | 1,3 | 3,8 | 3,8 |
| Schüttgewicht (g/l) | 535 | 485 | 470 | 430 | 400 |
| SiO₂-Gehalt (Gew.-%) | 99,9 | 99,9 | 99,9 | 99,9 | 99,9 |

### Beispiel 6

75 Gew.-% Aerosil 200
11,5 Gew.-% Methylcellulose
6 Gew.-% Mikrowachs
7,5 Gew.-% Polyethylenglycol
werden werden unter Zusatz von Wasser kompaktiert, bei 110 °C 16 Stunden getrocknet, zu einem rieselfähigen Pulver zerkleinert und mit einer Excenterpresse zu Preßlingen verformt. Die Rohtabletten werden 6 Stunden bei 750 °C getempert.

Die erhaltenen Preßlinge weisen folgende physikalischchemische Kenndaten auf:

| | |
|---|---|
| Tablettenform | Zylinder |
| Außendurchmesser x Höhe (mm) | 5 x 5 |
| BET-Oberfläche (m²/g) | 168 |
| Porenvolumen (ml/g) | 0,71 |
| Bruchfestigkeit (N) | 61 |
| Abrieb (Gew.-%) | 2,3 |
| Schüttgewicht (g/l) | 510 |
| SiO₂-Gehalt (Gew.-%) | 99,9 |

### Beispiele 7 und 8

71,5 Gew.-% Aerosil 300 (Beispiel 7) / Aerosil 130 (Beispiel 8)
13 Gew.-% Methylcellulose
7 Gew.-% Mikrowachs
8,5 Gew.-% Polyethylenglycol
werden unter Zusatz von Wasser kompaktiert, bei 110 °C 16 h getrocknet, zu einem rieselfähigen Pulver zerkleinert und mit einer Excenterpresse zu Preßlingen verformt. Die Rohtabletten werden 6 Stunden bei 750 °C calciniert.

Die erhaltenen Preßlinge weisen folgende physikalischchemische Kenndatne auf:

| **Beispiel** | **7** | **8** |
|---|---|---|
| Außendurchmesser x Höhe (mm) | 5 x 5 | 5 x 5 |
| BET-Oberfläche (m²/g) | 210 | 117 |
| Porenvolumen (ml/g) | 0,85 | 0,89 |
| Bruchfestigkeit (N) | 55 | 39 |
| Abrieb(Gew.-%) | 2,0 | 2,4 |
| Schüttgewicht (g/l) | 465 | 450 |
| SiO₂-Gehalt (Gew.-%) | 99,9 | 99,9 |

### Vergleichsbeispiel 1

Ein nicht erfindungsgemäßer Preßling (Katalysatorträger 350, Firma Degussa, mit 0,4 Gew.-% Mg (elementar), BET-Oberfläche 180 m²/g, Schüttdichte 490 g/l, gesamtes Porenvolumen 0,8 cm³/g, Tabletten von 6 mm Durchmesser und 5,5 mm Höhe) wird mit Phosphorsäure (60 Gew.-%) beladen und 41 Stunden in einer Hochdruckanlage bei einem Wasserdampfdruck von 15 bar und auf 350 °C belassen. Die Porenverteilung des gealterten Katalysators wird mit Hg-Porosimetrie bestimmt. Die gemessene Porenverteilung ist in Figur 1 grafisch dargestellt.

Die hydrothermal gealterten Träger weisen ein Maximum der Porenverteilung bei Porendurchmessern zwischen 20 und 30 µm auf. Den Anteil an Poren mit einem Durchmesser kleiner als 10 µm ist nahezu gleich null.

### Beispiel 9

Ein erfindungsgemäßer Träger nach Beispiel 3 (Mg-Gehalt < 50 Mikrogramm/g) wird mit Phosphorsäure (60 Gew.-%) beladen und 40 Stunden in einer Hochdruckanlage bei einem Wasserdampfdruck von 15 bar und 350 °C belassen. Die Porenverteilung des gealterten Katalysators wurde wiederum mit Hg-Porosimetrie bestimmt. Die Porenverteilung ist in Figur 2 grafisch dargestellt.

Das Maximum in der Porenverteilung liegt bei 30 µm. Im Vergleich zu dem im Vergleichsbeispiel 1 eingesetzten Katalysator weist der erfindungsgemäße Katalysator (Träger oder Katalysator) auch nach Alterung einen größeren Anteil an kleinen Poren mit einem Durchmesser unterhalb von 10 µm auf.

### Vergleichsbeispiel 2

Ein Katalysatorträger aus pyrogener Kieselsäure (BET-Oberfläche 180 m²/g, Schüttdichte 490 g/l, gesamtes Porenvolumen 0,8 cm³/g, Tabletten von 6 mm Durchmesser und 5,5 mm Höhe, mit 0,4 Gew.-% Mg (elementar)) wurde gemäß dem Beispiel 1 in der EP 0 519 435 für 14 Stunden mit 10 %iger Salzsäure bei Raumtemperatur in Kontakt gebracht und anschließend unter fließendem Wasser chloridfrei gewaschen und dann getrocknet.

Anschließend wurde ein Palladium-Gold-Kaliumacetat-Katalysator auf dem vorbehandelten Katalysatorträger hergestellt.

Die Konzentration der Imprägnierlösungen wurde so gewählt, daß der fertige Katalysator eine Konzentration von 0.55 Gew.-% Palladium, 0.25 Gew.-% Gold und 5,0 Gew.-% Kaliumacetat enthielt.

In einem ersten Schritt wurde der Träger zunächst mit einer basischen Lösung aus Natriumhydroxid in Wasser imprägniert. Das Volumen der wäßrigen NaOH-Lösung entsprach 50 Prozent der Wasseraufnahme des trockenen Trägers. Nach der Imprägnierung mit Natriumhydroxid wurde der Träger unmittelbar ohne Zwischentrocknung mit einer wäßrigen Edelmetalllösung aus Natriumpalladiumchlorid und Tetrachlorogoldsäure imprägniert, deren Volumen ebenfalls 50 Prozent der Wasseraufnahmekapazität des trockenen Trägermaterials entsprach.

Nach einer Wartezeit von 1,5 Stunden zwecks Hydrolyse der Edelmetallverbindungen wurden die Trägerteilchen chloridfrei gewaschen. Der Katalysator wurde getrocknet und bei 450°C in der Gasphase mit Formiergas reduziert. Danach wurde der Katalysator mit einer wäßrigen Kaliumacetat-Lösung imprägniert und erneut getrocknet. Die Trocknung wurde in der Gasphase mit Stickstoff durchgeführt.

Die Konzentration der basischen Lösung an Natriumhydroxid war so bemessen, daß sich auf den Trägerteilchen eine edelmetallhaltige Schale von < 1.0 mm ausbildete.

### Beispiel 10

Es wurde ein Palladium-Gold-Kaliumacetat-Katalysator wie in Vergleichsbeispiel 2 beschrieben auf dem erfindungsgemäßen Katalysatorträger nach Beispiel 3 (Mg-Gehalt < 50 Mikrogramm/g), jedoch mit einem Durchmesser von 6,0 mm und einer Höhe von 5,5 mm, hergestellt. Im Unterschied zum Vergleichsbeispiel 2 wurde jedoch auf die Vorbehandlung mit 10%iger Salzsäure verzichtet.

### Beispiel 11

Es wurde ein Palladium-Gold-Kaliumacetat-Katalysator nach Beispiel 10 auf dem erfindungsgemäßen Katalysatorträger nach Beispiel 5, jedoch mit den Abmessungen 8 x 5 x 3 mm und Facettenkanten, hergestellt.

### Anwendungsbeispiel 1

Aktivität und Selektivität der Katalysatoren aus dem Vergleichsbeispiel 2 und den Beispielen 10 und 11 wurden während einer Prüfung über die Dauer von bis zu 24 Stunden gemessen.

Die Katalysatoren wurden in einem mit Öl beheizten Strömungsrohrreaktor (Reaktorlänge 710 mm, Innendurchmesser 23,7 mm) bei Normaldruck und einer Raumgeschwindigkeit (GHSV) von 550 h⁻¹ mit der folgenden Gaszusammensetzung geprüft: 75 Vol.-% Ethen, 16,6 Vol.-% Essigsäure, 8,3 Vol.-% Sauerstoff. Die Katalysatoren wurden im Temperaturbereich von 120 bis 165°C, gemessen im Katalysatorbett, untersucht.

Die Reaktionsprodukte wurden im Ausgang des Reaktors mittels on-line Gaschromatographie analysiert. Als Maß für die Katalysatoraktivität wurde die Raum-Zeit-Ausbeute des Katalysators in Gramm Vinylacetat-Monomer pro Stunde und Kilogramm Katalysator (g VAM/(h x kg_{Kat.}) bestimmt.

Kohlendioxid, das insbesondere durch die Verbrennung von Ethen gebildet wird, wurde ebenfalls bestimmt und zur Beurteilung der Katalysatorselektivität herangezogen.

In Tabelle 1 sind die Untersuchungsergebnisse an den Katalysatoren aus dem Vergleichsbeispiel 2 und den Beispielen 10 und 11 dargestellt. Die Katalysatoraktivität und die Katalysatorselektivität des Katalysators nach Vergleichsbeispiel 2 wurden jeweils als 100 Prozent gesetzt.

**Tabelle 1:**

| Katalysator | Aktivität [g VAM/(h x kg_{Kat.}] in [%] von VB 2 | Selektivität CO₂ im Abgas in [Flächen-%] in [%] von VB 2 | Katalysatortemperatur [°C] |
|---|---|---|---|
| VB 2 | 100 | 100 | 159,6 |
| B 10 | 121,1 | 72,0 | 155,6 |
| | 122,7 | 96,8 | 161,1 |
| B 11 | 106,8 | 58,5 | 142,1 |
| | 124,9 | 103,9 | 154,1 |

Die Ergebnisse zeigen, daß die erfindungsgemäßen Katalysatoren bei vergleichbarer oder sogar verbesserter Selektivität eine deutlich höhere Aktivität aufweisen als der Vergleichskatalysator.

## Patentansprüche

1. Preßlinge auf Basis von pyrogen hergestelltem Siliciumdioxid mit den folgenden physikalisch-chemischen Kenndaten:
| | |
|---|---|
| Außendurchmesser | 0,8 - 20 mm |
| BET-Oberfläche | 30 - 400 m²/g |
| Porenvolumen | 0,5 - 1,3 ml/g |
| Bruchfestigkeit | 10 bis 250 N |
| Zusammensetzung | > 99,8 Gew.-% SiO₂ |
| Sonstige Bestandteile | < 0,2 Gew.-% |
| Abrieb | < 5 Gew.-% |
| Schüttgewicht | 350 - 750 g/l |

2. Verfahren zur Herstellung von Preßlingen auf Basis von pyrogen hergestelltem Siliciumdioxid mit folgenden physikalisch-chemischen Kenndaten:
| | |
|---|---|
| Außendurchmesser | 0,8 - 20 mm |
| BET-Oberfläche | 30 - 400 m²/g |
| Porenvolumen | 0,5 - 1,3 ml/g |
| Bruchfestigkeit | 10 bis 250 N |
| Zusammensetzung | > 99,8 Gew.-% SiO₂ |
| Sonstige Bestandteile | < 0,2 Gew.-% |
| Abrieb | < 5 Gew.-% |
| Schüttgewicht | 350 - 750 g/l |
**dadurch gekennzeichnet, daß** man pyrogen hergestelltes Siliciumdioxid mit Methylcellulose, Mikrowachs und Polyethylenglycol unter Zusatz von Wasser homogenisiert, bei einer Temperatur von 80 - 150 °C trocknet, gegebenenfalls zu einem Pulver zerkleinert, das Pulver zu Preßlingen verpreßt und während eines Zeitraumes von 0,5 bis 8 Stunden bei einer Temperatur von 400 bis 1200 °C tempert.

3. Verwendung der Preßlinge gemäß Anspruch 1 als Katalysator oder Katalysatorträger.

4. Trägerkatalysator für die Produktion von Vinylacetatmonomer enthaltend auf einem Träger katalytisch aktive Komponenten Palladium, Gold und Alkaliacetat, **dadurch gekennzeichnet, daß** der Träger ein Preßling gemäß Anspruch 1 ist.

5. Verfahren zur Herstellung des Trägerkatalysators nach Anspruch 4 für die Produktion von Vinylacetatmonomer durch Imprägnieren des Trägers mit einer basischen Lösung und einer Gold- und Palladiumsalze enthaltenden Lösung, wobei die Imprägnierung gleichzeitig oder nacheinander, mit oder ohne Zwischentrocknung erfolgt, Waschen des Trägers zur Entfernung gegebenenfalls vorhandener Chloridanteile und Reduzieren der auf dem Träger ausgefällten unlöslichen Verbindungen vor oder nach dem Waschen, Trocknen der so erhaltenen Katalysatorvorstufe, und Imprägnieren mit Alkaliacetaten oder Alkaliverbindungen, die sich unter den Reaktionsbedingungen bei der Produktion von Vinylacetatmonomer ganz oder teilweise in Alkaliacetate umwandeln, **dadurch gekennzeichnet, daß** der Träger ein Preßling gemäß Anspruch 1 ist.

6. Verwendung von Katalysatoren, welche eine Aktivkomponente enthalten, die auf einem Preßling gemäß Anspruch 1 aufgebracht ist, für katalytische Umsetzungen unter hydrothermalen Bedingungen.

7. Verwendung nach Anspruch 6 für die Hydratisierung von Olefinen.

8. Verwendung nach Anspruch 7, **dadurch gekennzeichnet, daß** als Aktivkomponente Phosphorsäure verwendet wird.

9. Verwendung nach Anspruch 7, **dadurch gekennzeichnet, daß** als Aktivkomponente eine Heteropolysäure aufgebracht ist.

10. Verwendung nach Anspruch 8 oder 9 für die Hydratisierung von Ethylen zur Herstellung von Ethanol und Diethylether.

11. Verwendung nach Anspruch 8 oder 9 für die Hydratisierung von Propylen zur Herstellung von Isopropanol.

## Claims

1. A moulding based on pyrogenically prepared silicon dioxide with the following physico-chemical characteristics:
| | |
|---|---|
| External diameter | 0.8 - 20 mm |
| BET surface area | 30 - 400 m²/g |
| Pore volume | 0.5 - 1.3 ml/g |
| Breaking strength | 10 to 250 N |
| Composition | > 99.8 wt.% SiO₂ |
| Other constituents | < 0.2 wt.% |
| Abrasion | < 5 wt.% |
| Bulk density | 350 - 750 g/l |

2. A process for preparing mouldings based on pyrogenically prepared silicon dioxide with the following physico-chemical characteristics:
| | |
|---|---|
| External diameter | 0.8-20 mm |
| BET surface area | 30 - 400 m²/g |
| Pore volume | 0.5 - 1.3 ml/g |
| Breaking strength | 10 to 250 N |
| Composition | > 99.8 wt.% SiO₂ |
| Other constituents | < 0.2 wt.% |
| Abrasion | < 5 wt.% |
| Bulk density | 350 - 750 g/l |
**characterised in that** pyrogenically prepared silicon dioxide is homogenised with methylcellulose, microwax and polyethylene glycol with the addition of water, dried at a temperature of 80 - 150°C, optionally crushed to a powder, the powder is compressed to form mouldings and conditioned for a period of 0.5 to 8 hours at a temperature of 400 to 1200°C.

3. Use of the mouldings according to Claim 1 as catalysts or catalyst supports.

4. A supported catalyst for the production of vinyl acetate monomer containing catalytically active components palladium, gold and alkali metal acetate on a support, **characterised in that** the support is a moulding according to Claim 1.

5. A process for preparing the supported catalyst according to Claim 4 for the production of vinyl acetate monomer, by impregnating the support with a basic solution and a solution containing a gold and a palladium salt, wherein impregnation takes place simultaneously or in sequence, with or without intermediate drying, washing the support to remove optionally present amounts of chloride and reducing the insoluble compounds precipitated on the support before or after washing, drying the catalyst precursor obtained in this way and impregnating with alkali metal acetate or alkali metal compounds which are converted entirely or partly to alkali metal acetates under the reaction conditions during the production of vinyl acetate monomer, **characterised in that** the support is a moulding according to Claim 1.

6. The use of catalysts which contain an active component which is applied to a moulding according to Claim 1 for catalytic reactions under hydrothermal conditions.

7. The use according to Claim 6 for the hydration of olefins.

8. The use according to Claim 7, **characterised in that** phosphoric acid is used as active component.

9. The use according to Claim 7, **characterised in that** a heteropolyacid is applied as active component.

10. The use according to Claim 8 or 9 for the hydration of ethylene to prepare ethanol and diethyl ether.

11. The use according to Claim 8 or 9 for the hydration of propylene to prepare isopropanol.

## Revendications

1. Comprimés à base de dioxyde de silicium préparés par pyrogénation ayant les caractéristiques physico-chimiques suivantes :
| | |
|---|---|
| Diamètre externe | 0,8 - 20 mm |
| Surface BET | 30 - 400 m²/g |
| Volume des pores | 0,5 - 1,3 ml/g |
| Résistance à la rupture | de 10 à 250 N |
| Composition | > 99,8 % en poids de SiO₂ |
| Autres constituants | < 0,2 % en poids |
| Abrasion | < 5 % en poids |
| Densité apparente | 350 - 750 g/l |

2. Procédé de préparation de comprimés à base de dioxyde de silicium préparé par pyrogénation ayant les caractéristiques physico-chimiques suivantes :
| | |
|---|---|
| Diamètre externe | 0,8 - 20 mm |
| Surface BET | 30 - 400 m²/g |
| Volume des pores | 0,5 - 1,3 ml/g |
| Résistance à la rupture | 10 - 250 N |
| Composition | > 99,8 % en poids de SiO₂ |
| Autres constituants | < 0,2 % en poids |
| Abrasion | < 5 % en poids |
| Densité apparente | 350 - 750 g/l |
**caractérisé en ce qu'**
on homogénéise le dioxyde de silicium préparé par pyrogénation avec de la méthylcellulose, de la microcire et du polyéthylèneglycol tout en ajoutant de l'eau, séché à une température de 80 à 150°C, on broie le cas échéant en une poudre, comprime la poudre en comprimés et pendant un laps de temps de 0,5 à 8 heures recuit à une température de 400 à 1200°C.

3. Utilisation des comprimés conformément à la revendication 1, comme catalyseur ou support de catalyseur.

4. Catalyseur sur support pour la production de monomère d'acétate de vinyle contenant des composants actifs catalytiquement sur un support, le palladium, l'or et l'acétate de métal alcalin,
**caractérisé en ce que**
le support est un comprimé conformément à la revendication 1.

5. Procédé de préparation du catalyseur sur support, selon la revendication 4, pour la production du monomère d'acétate de vinyle par imprégnation du support avec une solution basique et une solution contenant des sels d'or et de palladium, selon lequel l'imprégnation s'effectue simultanément ou consécutivement avec ou sans séchage intermédiaire, par lavage du support pour l'élimination des fractions de chlorures éventuellement présentes et réduction des composés insolubles précipités sur le support avant ou après le lavage, par séchage du stade précurseur de catalyseur ainsi obtenu et imprégnation avec des acétates de métal alcalin ou des composés de métal alcalin qui se convertissent dans les conditions de la réaction lors de la production de monomère d'acétate de vinyle totalement ou partiellement en acétate de métal alcalin,
**caractérisé en ce que**
le support est un comprimé conformément à la revendication 1.

6. Utilisation de catalyseur qui renferment un composant actif qui est appliqué sur un comprimé conformément à la revendication 1, pour des réactions catalytiques dans des conditions hydrothermiques.

7. Utilisation selon la revendication 6 pur l'hydratation d'oléfines.

8. Utilisation selon la revendication 7,
**caractérisée en ce qu'**
on utilise comme composant actif de l'acide phosphorique.

9. Utilisation selon la revendication 7,
**caractérisée en ce qu'**
on applique comme composant actif un hétéropolyacide.

10. Utilisation selon la revendication 8 ou la revendication 9, pour l'hydratation de l'éthylène en vue de la production d'éthanol et d'éther diéthylique.

11. Utilisation selon l'une quelconque des revendications 8 ou 9, pour l'hydratation du propylène en vue de la production d'isopropanol.
